# EUROPEAN PATENT SPECIFICATION

(11) **EP 2 519 234 B1**
(45) Date of publication and mention of the grant of the patent: **28.06.2017**
(21) Application number: 10841613.2
(22) Date of filing: 23.12.2010
(51) Int. Cl.: A61K 31/23, A61P 25/28

(54) **ANAPLEROTIC THERAPY FOR ALZHEIMER'S DISEASE**
ANAPLEROTISCHE THERAPIE FÜR MORBUS ALZHEIMER
THÉRAPIE ANAPLÉROTIQUE POUR LA MALADIE D'ALZHEIMER

(30) Priority: 30.12.2009 US 291276 P
(43) Date of publication of application: 07.11.2012
(73) Proprietor: Baylor Research Institute, Dallas, TX 75204 (US)
(72) Inventor: BOTTIGLIERI, Teodoro, G., Dallas TX 75225 (US); ROE, Charles, R., Rockwall TX 75087 (US)
(74) Representative: Moore, Jacqueline Frances
(86) International application number: PCT/US2010/062054
(87) International publication number: WO 2011/082111

(56) References cited:
- WO-A1-2008/068230
- WO-A2-2004/103307
- WO-A2-2009/018478
- DE-A1- 3 032 300
- US-A- 4 753 963
- US-A1- 2006 004 099
- US-A1- 2008 132 571
- US-B1- 6 835 750
- HENDERSON ET AL: "Ketone Bodies as a Therapeutic for Alzheimer's Disease", NEUROTHERAPEUTICS, ELSEVIER INC, US, vol. 5, no. 3, July 2008 (2008-07), pages 470-480, XP023438787, ISSN: 1933-7213, DOI: 10.1016/J.NURT.2008.05.004 [retrieved on 2008-07-12]

## Description

### TECHNICAL FIELD OF THE INVENTION

The present invention relates in general to the field of treatment agents for Alzheimer's disease (AD) and aging, and more particularly to the use of a ketogenic diet comprising odd-chain triglycerides for improving cognition, increasing circulating ketone bodies, and for reducing amyloid-**β(Aβ)** deposition in AD patients.

### BACKGROUND OF THE INVENTION

Without limiting the scope of the invention, its background is described in connection with the use of an anaplerotic diet for the treatment and the mitigation of symptoms associated with neurodegenerative conditions including AD.

United States Patent No. 6,335,361 issued to Nathan (2002) discloses methods to treat cognition disorders, particularly those associated with aging. The method comprises administering a combination of a carnitine and an oxidant. Preferably the oxidant is thioctic acid. Preferably 0.12 grams to 3 grams of carnitine (particularly ALC) and 0.12 and 1.5 grams of R-a-lipoic acid are administered. Optionally, coenzyme Q and/or creatine also are administered. Preferably 10 mg to 500 mg/day of coenzyme Q10 and 1 to 30 grams/day of creatine are administered. The same method can be used to treat cognition deficits associated with carbon monoxide poisoning, mild traumatic brain injury, Type 2 diabetes mellitus, obsessive-compulsive disorder, environmental toxin exposure, and other conditions.

United States Patent Application No. 20080287372 (Henderson, 2008) relates to the field of therapeutic agents for the treatment of Age-Associated Memory Impairment (AAMI). In particular, the present invention utilizes compositions comprising at least one compound capable of elevating ketone body concentrations in a mammal (e.g., ketogenic compounds), administered in an amount effective for treatment or prevention of loss of cognitive function caused by reduced neuronal metabolism in AAMI. In one embodiment, the composition includes medium chain triglycerides (MCT). In another embodiment, the compositions are administered in the presence of carbohydrate. The present invention also relates to oral dosage forms, in particular, a nutritional drink comprising at least one compound capable of elevating ketone body concentrations in a mammal.

United States Patent Application No. 20090253781 (Veech, 2009) discloses compositions comprising ketone bodies and/or their metabolic precursors are provided that are suitable for administration to humans and animals and which have the properties of, inter alia, (i) increasing cardiac efficiency, particularly efficiency in use of glucose, (ii) for providing energy source, particularly in diabetes and insulin resistant states and (iii) treating disorders caused by damage to brain cells, particularly by retarding or preventing brain damage in memory associated brain areas such as found in Alzheimer's and similar conditions. These compositions may be taken as nutritional aids, for example for athletes, or for the treatment of medical conditions, particularly those associated with poor cardiac efficiency, insulin resistance and neuronal damage. The invention further provides methods of treatment and novel esters and polymers for inclusion in the compositions of the invention.

### SUMMARY OF THE INVENTION

There is herein described the use of an anaplerotic diet for the therapy of neurological and neurodegenerative conditions in human subjects including AD and aging.

In accordance with the invention, there is provided a formulation for use in treating an adult patient suffering from Alzheimer's Disease (AD) comprising the steps of:
administering the formulation to the patient in a quantity sufficient to treat of alleviate the symptoms of Alzheimer's Disease (AD), wherein the formulation comprises triheptanoin.
wherein, the R1, R2, and R3 are esterified to the glycerol backbone are each independently fatty acids comprising odd numbered carbon chains having 5 to 15 carbon atoms, and one or more optional additives selected from the group consisting of flavoring agents, vitamins, mineral supplements, protein supplements, coloring agents, and preservatives. In one aspect, the R1, R2, and R3 carbon chains are five carbons in length selected from pentanoin, , pentanoylcarnitine, n-pentadecanoic acid, five carbon fatty acid precursors, and derivatives thereof. In one aspect, the odd-chain triglyceride is tripentanoin. In one aspect, the R1, R2, and R3 carbon chains are seven carbons in length. In another aspect, the odd-chain triglyceride is triheptanoin. In another aspect, the neurodegenerative disorder is Alzheimer's disease (AD) or aging.

Yet another embodiment of the present invention is a method of treating an adult patient suffering from Alzheimer's disease (AD) comprising the steps of: identifying the adult patient in need of treatment against the AD; and administering a formulation of triheptanoin to the patient enterally or parenterally in a quantity sufficient to treat or alleviate the symptoms of the AD. In one aspect, the formulation comprises one or more optional additives selected from the group consisting of flavoring agents, vitamins, mineral supplements, protein supplements, coloring agents, and preservatives. In another aspect, the composition is administered to a subject and increases a level of one or more circulating ketone bodies in the blood of the human subject.

Another embodiment of the present invention is a dietary composition and/or a diet for providing a high fat, a low carbohydrate diet to a human subject comprising: one or more odd- chain triglycerides having the general formula: 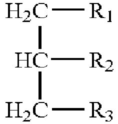 wherein, the R1, R2, and R3 are esterified to the glycerol backbone are each independently fatty acids comprising odd numbered carbon chains having 5 to 15 carbon atoms; and one or more optional additives selected from the group consisting of flavoring agents, vitamins, mineral supplements, protein supplements, coloring agents, and preservatives. In one aspect, the R1, R2, and R3 carbon chains are five carbons in length selected from pentanoin, pentanoylcarnitine, n-pentadecanoic acid, five carbon fatty acid precursors, and derivatives thereof. In another aspect, the R1, R2, and R3 carbon chains are seven carbons in length. In another aspect, the odd-chain fatty acid compound is tripentanoin or triheptanoin. In another aspect, the human subject is a healthy human subject or a In one aspect, the formulation comprises one or more optional additives selected from the group consisting of flavoring agents, vitamins, mineral
supplements, protein supplements, coloring agents, and preservatives. In another aspect, upon administration the composition increases a level of one or more circulating ketone bodies in the blood of the human subject. In yet another aspect, the formulation is adapted for parenteral, enteral, intravenous, or intramuscular administration.

### BRIEF DESCRIPTION OF THE DRAWINGS

For a more complete understanding of the features and advantages of the present invention, reference is now made to the detailed description of the invention along with the accompanying figures and in which:
**FIG. 1** is a schematic of the Model 1 study to determine efficacy of C5-TG in a transgenic mouse model that over-express APP and Aβ;
**FIG. 2** is a schematic of the Model 2 study to determine efficacy of C5-TG in a transgenic mouse model that over-expresses microtubule associated phosphorylated Tau (MAPT) in the absence of other factors including Aβ; and
**FIG. 3** is a schematic of the Model 3 study to determine cognitive functions and locomotive activity in aged mice.

### DETAILED DESCRIPTION OF THE INVENTION

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

The present disclosure describes an anaplerotic diet therapy for the treatment of AD and other neurodegenerative conditions. The high fat, adequate protein, and low carbohydrate diet increases circulating ketone bodies, reduces amyloid-β(Aβ) deposition in AD patients, thereby improving cognition and locomotive skills.

As used herein, the terms "subject" or "patient" are intended to include living organisms that may have one or more conditions referred to herein. Examples of subjects include humans, monkeys, horses, cows, sheep, goats, dogs, cats, mice, rats, and transgenic species thereof. Other examples of subjects include experimental animals such as mice, rats, dogs, cats, goats, sheep, pigs, and cows. A subject can be a human suffering from, or suspected of having, against a neurological or a neurodegenerative disorder.

As used herein, the phrases "therapeutically effective dosage" or "therapeutically effective amount" is an amount of a compound or mixtures of compounds, such as the odd-chain fatty acids and precursors or derivatives thereof, that reduce the amount of one or more symptoms of the condition in the infected subject by at least about 20%, at least about 40%, even more at least about 60%, 80% or even 100% relative to untreated subjects with a neurological or a neurodegenerative disorder. Active compounds are administered at a therapeutically effective dosage sufficient to treat a condition associated with a condition in a subject. For example, the efficacy of a compound can be evaluated in patients or animal model systems that may be predictive of efficacy in treating the disease in humans or animals.

As used herein the term, "odd-chain fatty acids" is used to describe fats and oils in foods are made up of basic units called fatty acids. In the body, these typically travel in three's as fatty acid chains attached to glycerol, forming a triglyceride. An odd-chain fatty acid that is attached to glycerol is described herein as an odd-chain triglyceride. Both the odd-chain fatty acid and the odd-chain triglyceride are described herein and are often used interchangeably. For example, when referring to an odd-chain fatty acid it is possible to substitute with, or provide as, the odd-chain triglyceride and vice verse.

Based on their chemical structure, fatty acids are classified into 3 major categories: monounsaturated, polyunsaturated, or saturated fats. The oils and fats that people and animals eat are nearly always mixtures of these 3 types of fatty acids, with one type predominating. Two specific types of polyunsaturated fatty acids, linoleic and alpha-linoleic, are called essential fatty acids. They must be present in the diet in adequate amounts because they are considered necessary for proper nutrition and health. Linoleic acid (LA) is an omega-6 fatty acid and is found in many oils, e.g., com, safflower, soybean and sunflower, whole grains and walnuts. Alpha-linoleic acid (ALA) is a plant precursor of docosahexanoic acid (DHA). Sources of ALA include seaweeds and green leaves of plants (in very small amounts), soybeans, walnuts, butternuts, some seeds (flax, chia, hemp, canola) and the oils extracted from these foods.

As used herein, the term "nutritionally effective amount" is used to mean the amount of odd-chain fatty acids and/or odd-chain triglycerides that will provide a beneficial nutritional effect or response in a mammal. For example, as with a nutritional response to vitamin- and mineral-containing dietary supplements varies from mammal to mammal, it should be understood that nutritionally effective amounts of the odd-chain fatty acids will vary. Thus, while one mammal may require a particular profile of vitamins and minerals present in defined amounts, another mammal may require the same particular profile of vitamins and minerals present in different defined amounts.

When provided as a dietary supplement or additive, the odd-chain fatty acids and/or odd-chain triglycerides has been prepared and administered to mammals in powdered, reconstitutable powder, liquid-solid suspension, liquid, capsule, tablet, caplet, lotion and cream dosage forms. The skilled artisan in the science of formulations can use the odd-chain fatty acids disclosed herein as a dietary supplement that may be formulated appropriately for, e.g., irrigation, ophthalmic, otic, rectal, sublingual, transdermal, buccal, vaginal, or dermal administration. Thus, other dosage forms such as chewable candy bar, concentrate, drops, elixir, emulsion, film, gel, granule, chewing gum, jelly, oil, paste, pastille, pellet, shampoo, rinse, soap, sponge, suppository, swab, syrup, chewable gelatin form, chewable tablet and the like, can be used.

Due to varying diets among people, the dietary odd-chain fatty acids may be administered in a wide range of dosages and formulated in a wide range of dosage unit strengths. It should be noted that the dosage of the dietary supplement can also vary according to a particular ailment or disorder that a mammal is suffering from when taking the supplement. For example, a person suffering from chronic fatigue syndrome or fibromyalgia will generally require a dose different than an athlete that is wanting to attain a nutritional benefit or obtain an increase in mental focus. An appropriate dose of the dietary supplement can be readily determined by monitoring patient response, i.e., general health, to particular doses of the supplement. The appropriate doses of the supplement and each of the agents can be readily determined in a like fashion by monitoring patient response, i.e., general health to particular doses of each.

The odd-chain fatty acids may be administered simultaneously or sequentially in one or a combination of dosage forms. While it is possible and even likely that the present dietary supplement will provide an immediate overall health benefit, such benefit may take days, weeks or months to materialize. Nonetheless, the present dietary odd-chain fatty acid supplement will provide a beneficial nutritional response in a mammal consuming it.

The odd-chain fatty acids may be administered, e.g., orally or by subcutaneous, intravenous, intraperitoneal, etc., administration (e.g. by injection). Depending on the route of administration, the active compound may be neutralized, made miscible, at least partially or fully water-soluble or even coated in a material to protect the odd-chain fatty acids from the action of bases, acids, enzymes or other natural conditions that may interfere with their effectiveness, uptake or metabolic use.

To administer the therapeutic compound by other than parenteral administration, it may be necessary to coat the compound with, or co-administer the compound with, a material to prevent its inactivation. For example, the therapeutic compound may be administered to a subject in an appropriate carrier, for example, emulsifiers, liposomes, or a diluent. Pharmaceutically acceptable diluents include saline and aqueous buffer solutions. The therapeutic odd-chain fatty acids may be dispersed in glycerol, liquid polyethylene glycols, and mixtures thereof and in oils. Under ordinary conditions of storage and use, these preparations may contain a preservative to prevent the growth of microorganisms.

Pharmaceutical compositions that include the odd-chain fatty acids of the disclosure suitable for injectable use may include sterile aqueous solutions, dispersions and sterile powders for the extemporaneous preparation of sterile injectable solutions or dispersion. In all cases, the composition must be sterile and must be fluid to the extent that easy syringability exists. It must be stable under the conditions of manufacture and storage and must be preserved against the contaminating action of microorganisms such as bacteria and fungi.

The odd-chain fatty acids may be provided with a carrier in a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, sodium chloride, or polyalcohols such as mannitol and sorbitol, in the composition. Including in the composition an agent, which delays absorption, for example, aluminum monostearate or gelatin can bring about prolonged absorption of the injectable compositions.

The odd-chain fatty acids may be provided in one or more controlled sizes and characteristics with one or more water-soluble polymers depending on the size and structural requirements of the patient, e.g., the particles may be small enough to traverse blood vessels when provided intravenously. Either synthetic or naturally occurring polymers may be used, and while not limited to this group, some types of polymers that might be used are polysaccharides (e.g. dextran, ficoll), proteins (e.g. poly-lysine), poly(ethylene glycol), or poly(methacrylates). Different polymers, because of their different size and shape, will produce different diffusion characteristics for the odd-chain fatty acids in the target tissue or organ.

Sterile injectable solutions can be prepared by incorporating the therapeutic compound in the required amount in an appropriate solvent with one or a combination of ingredients enumerated above, as required, followed by filtered sterilization. Generally, dispersions are prepared by incorporating the therapeutic compound into a sterile carrier, which contains a basic dispersion medium and the required other ingredients from those enumerated above. In the case of sterile powders for the preparation of sterile injectable solutions, methods of preparation include: vacuum drying, spray freezing, freeze-drying and the like, which yield a powder of the active ingredient (i.e., the therapeutic compound) plus any additional desired ingredient from a previously sterile-filtered solution thereof.

The odd-chain fatty acids can be orally administered, for example, with an inert diluent or an assimilable edible carrier. The therapeutic compound and other ingredients may also be enclosed in a hard or soft shell gelatin capsule, compressed into tablets, or incorporated directly into the subject's diet. The odd-chain fatty acids may be incorporated with one or more excipients for use in, e.g., ingestible tablets, buccal tablets, troches, capsules, elixirs, suspensions, syrups, wafers, and the like. The amount of odd-chain fatty acids in the compositions and preparations may, of course, be varied depending on, e.g., the age, weight, gender, condition, disease and course of treatment of the individual patient. Pediatric doses are likely to differ from adult doses as will be known to the skilled artisan. The amount of the therapeutic compound in such therapeutically useful compositions is such that a suitable dosage will be obtained.

A dosage unit for use with the odd-chain fatty acids disclosed herein may be a single compound or mixtures thereof with other compounds, e.g., amino acids, nucleic acids, vitamins, minerals, pro-vitamins and the like. The compounds may be mixed together, form ionic or even covalent bonds. For pharmaceutical purposes the odd-chain fatty acids (e.g., C5, C7, C9, C11, C13 and/or C15) of the present disclosure may be administered in oral, intravenous (bolus or infusion), intraperitoneal, subcutaneous, or intramuscular form, all using dosage forms well known to those of ordinary skill in the pharmaceutical arts. Depending on the particular location or method of delivery, different dosage forms, e.g., tablets, capsules, pills, powders, granules, elixirs, tinctures, suspensions, syrups, and emulsions may be used to provide the odd-chain fatty acids to a patient in need of therapy that includes a number of conditions, e.g., polysaccharide storage diseases, fatigue, low energy, wasting and the like. The odd-chain fatty acids may also be administered as any one of known salt forms.

The total daily amount of odd-chain fatty acids will vary depending on the condition and needs of a patient. For example, the odd-chain fatty acids may be provided as a supplemental source of immediate, short-term, mid-term or long-term energy and may be provided in formulations that are immediately available, slow release or extended release. The dosage amount may be measured in grams per day, as a percentage of kCalories consumed in a day, as a percentage of the total daily caloric intake, as part of a fixed, a modified or a diet that changes over time. For example, a patient may need immediate intervention that "spikes" the amount of odd-chain fatty acids to approach or reach ketosis. These "ketogenic" odd-chain fatty acids will then be varied to not have other side effects, e.g., start with 40% of total caloric intake per day and then reduced over time as the patient's condition, symptoms, clinical course and/or metabolic conditions improves. The range of percentage caloric intake may vary from between about 0.01, 0.1, 1, 2, 5, 10, 15, 20, 22, 25, 30, 35, 40 or even higher percent, which may include one or more of the odd-chain fatty acids (e.g., C5, C7, C9, C11, C13 and/or C15 (available from, e.g., Sassol, Germany). One way to measure the effect and/or dosing of the odd-chain fatty acids is to measure the amount that is detectable in body solids or fluids, e.g., biopsies and blood, respectively. A wide variety of odd-chain fatty acids metabolites may be detected from multiple sources, e.g., urine, tears, feces, blood, sweat, breath and the like.

For example, when using C7 as the source of odd-chain fatty acids these can be provided in the form of a triglyceride, e.g., tri-heptanoin. The triglyceride triheptanoin is provided in a concentration sufficient to provide a beneficial effect is most useful in this aspect of the present invention. The seven-carbon fatty acid may be provided, e.g.:

| | | |
|---|---|---|
| Infants | 1-4 g/kg | 35% kcalories |
| Children | 3-4 g/kg | 33-37% kcalories |
| Adolescent | 1-2 g/kg | 35% kcalories |
| Adults | 0.1-2g/kg | 35% kcalories |

Goals have been set using 4 g/kg (within ideal body weight (IBW) range) for infants, children, and some adolescents. Goals have been set using 2 g/kg (within IBW range) for adolescents. Goals have been set using 2 g/kg (within IBW range) for adults; but toleration is 1 - 1.2 g per kg (which is 35% kcal of estimated needs).

The odd-chain fatty acids are typically administered in admixture with suitable pharmaceutical salts, buffers, diluents, extenders, excipients and/or carriers (collectively referred to herein as a pharmaceutically acceptable carrier or carrier materials) selected based on the intended form of administration and as consistent with conventional pharmaceutical practices. Depending on the best location for administration, the odd-chain fatty acids may be formulated to provide, e.g., maximum and/or consistent dosing for the particular form for oral, rectal, topical, intravenous injection or parenteral administration. While the odd-chain fatty acids may be administered alone or pure, they may also be provided as stable salt form mixed with a pharmaceutically acceptable carrier. The carrier may be solid or liquid, depending on the type and/or location of administration selected.

Techniques and compositions for making useful dosage forms are described in one or more of the following references: Ansel, Introduction to Pharmaceutical Dosage Forms 2nd Edition (1976); Remington's Pharmaceutical Sciences, 17th ed. (Mack Publishing Company, Easton, Pa., 1985); Advances in Pharmaceutical Sciences (David Ganderton, Trevor Jones, Eds., 1992); Advances in Pharmaceutical Sciences Vol 7. (David Ganderton, Trevor Jones, James McGinity, Eds., 1995); Aqueous Polymeric Coatings for Pharmaceutical Dosage Forms (Drugs and the Pharmaceutical Sciences, Series 36 (James McGinity, Ed., 1989); Pharmaceutical Particulate Carriers: Therapeutic Applications: Drugs and the Pharmaceutical Sciences, Vol 61 (Alain Rolland, Ed., 1993); Drug Delivery to the Gastrointestinal Tract (Ellis Horwood Books in the Biological Sciences. Series in Pharmaceutical Technology; J. G. Hardy, S. S. Davis, Clive G. Wilson, Eds.); Modem Pharmaceutics Drugs and the Pharmaceutical Sciences, Vol40 (GilbertS. Banker, Christopher T. Rhodes, Eds.), and the like.

Odd-chain fatty acids may be administered in the form of an emulsion and/or liposome, e.g., small unilamellar vesicles, large unilamallar vesicles and multilamellar vesicles, whether charged or uncharged. Liposomes may include one or more: phospholipids (e.g., cholesterol), stearylamine and/or phosphatidylcholines, mixtures thereof, and the like. Examples of emulsifiers include: Imwitor 370, Imwitor 375, Imwitor 377, Imwitor 380 and Imwitor 829.

The odd-chain fatty acid vesicles may also be coupled to one or more soluble, biodegradable, bioacceptable polymers as drug carriers or as a prodrug. Such polymers may include: polyvinylpyrrolidone, pyran copolymer, polyhydroxylpropylmethacrylamide-phenol, polyhydroxyethylaspartamidephenol, or polyethyleneoxide-polylysine substituted with palmitoyl residues, mixtures thereof, and the like. Furthermore, the vesicles may be coupled one or more biodegradable polymers to achieve controlled release of the odd-chain fatty acids. Biodegradable polymers include, e.g., polylactic acid, polyglycolic acid, copolymers of polylactic and polyglycolic acid, polyepsilon caprolactone, polyhydroxy butyric acid, polyorthoesters, polyacetals, polydihydropyrans, polycyanoacylates, and crosslinked or amphipathic block copolymers of hydrogels, mixtures thereof, and the like.

In one embodiment, gelatin capsules (gelcaps) may include the odd-chain fatty acid in its native state. For oral administration in a liquid dosage form, the oral drug components may be combined with any oral, non-toxic, pharmaceutically acceptable inert carrier such as an emulsifier, a diluent or solvent (e.g., ethanol), glycerol, water, and the like. Examples of suitable liquid dosage forms include oily solutions or suspensions in water, pharmaceutically acceptable fats and oils, alcohols or other organic solvents, including esters, emulsions, syrups or elixirs, suspensions, solutions and/or suspensions reconstituted from non-effervescent granules and even effervescent preparations reconstituted from effervescent granules. Such liquid dosage forms may contain, for example, suitable solvents, preservatives, emulsifying agents, suspending agents, diluents, sweeteners, thickeners, and melting agents, mixtures thereof, and the like.

Liquid dosage forms for oral administration may also include coloring and flavoring agents that increase patient acceptance and therefore compliance with a dosing regimen. In general, water, a suitable oil, saline, aqueous dextrose (e.g., glucose, lactose and related sugar solutions) and glycols (e.g., propylene glycol or polyethylene glycols) may be used as suitable carriers for parenteral solutions. Solutions for parenteral administration include generally, a water-soluble salt of the active ingredient, suitable stabilizing agents, and if necessary, buffering salts. Antioxidizing agents such as sodium bisulfite, sodium sulfite and/or ascorbic acid, either alone or in combination, are suitable stabilizing agents. Citric acid and its salts and sodium EDTA may also be included to increase stability. In addition, parenteral solutions may include pharmaceutically acceptable preservatives, e.g., benzalkonium chloride, methyl- or propyl-paraben, and/or chlorobutanol. Suitable pharmaceutical carriers are described in multiple editions of Remington's Pharmaceutical Sciences, Mack Publishing Company, a standard reference text in this field.

For direct delivery to the nasal passages, sinuses, mouth, throat, esophagus, trachea, lungs and alveoli, the odd-chain fatty acids may also be delivered as an intranasal form via use of a suitable intranasal vehicle. For dermal and transdermal delivery, the odd-chain fatty acids may be delivered using lotions, creams, oils, elixirs, serums, transdermal skin patches and the like, as are well known to those of ordinary skill in that art. Parenteral and intravenous forms may also include pharmaceutically acceptable salts and/or minerals and other materials to make them compatible with the type of injection or delivery system chosen, e.g., a buffered, isotonic solution.

To the extent that the odd-chain fatty acids may be made into a dry powder or form, they may be included in a tablet. Tablets will generally include, e.g., suitable binders, lubricants, disintegrating agents, coloring agents, flavoring agents, flow-inducing agents and/or melting agents. For example, oral administration may be in a dosage unit form of a tablet, gelcap, caplet or capsule, the active drug component being combined with a non-toxic, pharmaceutically acceptable, inert carrier such as lactose, gelatin, agar, starch, sucrose, glucose, methyl cellulose, magnesium stearate, dicalcium phosphate, calcium sulfate, mannitol, sorbitol, mixtures thereof, and the like. Suitable binders include: starch, gelatin, natural sugars (e.g., glucose or beta-lactose), com sweeteners, natural and synthetic gums (e.g., acacia, tragacanth or sodium alginate), carboxymethylcellulose, polyethylene glycol, waxes, and the like. Lubricants for use with the invention may include: sodium oleate, sodium stearate, magnesium stearate, sodium benzoate, sodium acetate, sodium chloride, mixtures thereof, and the like. Disintegrators may include: starch, methyl cellulose, agar, bentonite, xanthan gum, mixtures thereof, and the like.

Capsules. Capsules may be prepared by filling standard two-piece hard gelatin capsules each with 10 to 500 milligrams of powdered active ingredient, 5 to 150 milligrams of lactose, 5 to 50 milligrams of cellulose and 6 milligrams magnesium stearate.

Soft Gelatin Capsules. The odd-chain fatty acids may be dissolved in an oil, e.g., a digestible oil such as soybean oil, cottonseed oil or olive oil. Non-digestible oils may also be used to have better control over the total caloric intake provided by the oil. The active ingredient is prepared and injected by using a positive displacement pump into gelatin to form soft gelatin capsules containing, e.g., 100-500 milligrams of the active ingredient. The capsules are washed and dried.

Tablets. A large number of tablets are prepared by conventional procedures so that the dosage unit was 100-500 milligrams of active ingredient, 0.2 milligrams of colloidal silicon dioxide, 5 milligrams of magnesium stearate, 50-275 milligrams of microcrystalline cellulose, 11 milligrams of starch and 98.8 milligrams of lactose. Appropriate coatings may be applied to increase palatability or delay absorption.

To provide an effervescent tablet, appropriate amounts of, e.g., monosodium citrate and sodium bicarbonate, are blended together and then roller compacted, in the absence of water, to form flakes that are then crushed to give granulates. The granulates are then combined with the active ingredient, drug and/or salt thereof, conventional beading or filling agents and, optionally, sweeteners, flavors and lubricants.

Injectable solution. A parenteral composition suitable for administration by injection is prepared by stirring sufficient active ingredient in deionized water and mixed with, e.g., up to 10% by volume propylene glycol, salts and/or water to deliver a composition, whether in concentrated or ready-to-use form. Given the nature of the odd-chain fatty acids (alone, partially or fully-soluble in water) the amount and final concentration of the odd-chain fatty acids may be varied such that the liquid may be provided intravenously using syringes and/or standard intravenous liquids or fluids. The solution will generally be made isotonic with sodium chloride and sterilized using, e.g., ultrafiltration.

Suspension. An aqueous suspension is prepared for oral administration so that each 5 ml contain 100 mg of finely divided active ingredient, 200 mg of sodium carboxymethyl cellulose, 5 mg of sodium benzoate, 1.0 g of sorbitol solution, U.S.P., and 0.025 ml of vanillin.

Mini-tablets. For mini-tablets, the active ingredient is compressed into a hardness in the range 6 to 12 Kp. The hardness of the final tablets is influenced by the linear roller compaction strength used in preparing the granulates, which are influenced by the particle size of, e.g., the monosodium hydrogen carbonate and sodium hydrogen carbonate. For smaller particle sizes, a linear roller compaction strength of about 15 to 20 KN/cm may be used.

Kits. The present disclosure also includes pharmaceutical kits useful, for example, for providing an immediate source of alternative cellular energy, e.g., before, during or after surgery. The dosage will generally be prepared sterile and ready-to-use, e.g., one or more containers that may be broken (e.g., sealed glass ampoules), pierced with a syringe for immediate administration or even a pressurized container. Such kits may further include, if desired, one or more of various conventional pharmaceutical kit components, such as, for example, containers with one or more pharmaceutically acceptable diluents, carriers, additional containers, etc., as will be readily apparent to those skilled in the art. Printed instructions, either as inserts or as labels, indicating quantities of the components to be administered, guidelines for administration, and/or guidelines for mixing the components, may also be included in the kit.

Pharmaceutical Dosage Forms. The odd-chain fatty acids may be provided in liquid form or may also be provided in a capsule, gelcap or other encapsulated form. Generally, one composition is prepared by adding, e.g., half of the Kaolin clay or other carrier into the blended followed by addition of a first active salt form, e.g., the salt form that is less soluble in the final liquid suspension, e.g., as an emulsion in water. This process is particularly suitable for very large mixtures, e.g., 500, 1,000, 3,000 or even 5,000 liters.

One particular method of delivery of the odd-chain fatty acids is in a tablet, capsule or gelcap that is coated for enteric delivery. Enteric coating relates to a mixture of pharmaceutically acceptable excipient(s) that is/are applied to, combined with, mixed with or
otherwise added to a carrier to deliver the medicinal content, in this case one or more odd-chain fatty acids (e.g., C5, C7, C9, C11, C13 and/or C15, mixtures and combinations thereof) through the stomach unaltered for delivery into the intestines. The coating may be applied to a compressed or molded or extruded tablet, a gelatin capsule, and/or pellets, beads, granules or particles of the carrier or composition. The coating may be applied through an aqueous dispersion or after dissolving in appropriate solvent. Additional additives and their levels, and selection of a primary coating material or materials will depend on the following properties: resistance to dissolution and disintegration in the stomach; impermeability to gastric fluids and drug/carrier/enzyme while in the stomach; ability to dissolve or disintegrate rapidly at the target intestine site; physical and chemical stability during storage; non-toxicity; easy application as a coating (substrate friendly); and economical practicality. Methods for enteric coating are well known in the art.

Remington's Pharmaceutical Sciences, discloses that enteric polymer carries generally include carboxyl groups and hydrophobic groups in the molecule and the enteric polymer is dissolved in a solvent having a specific pH value through the dissociation of the carboxyl groups. For instance, commercially available hydroxypropylmethyl cellulose acetate succinate is a derivative of hydroxypropylmethyl cellulose, which is substituted with carboxyl groups (succinoyl groups) and hydrophobic groups (acetyl groups). Alginic acid, sodium alginate other natural materials may also be used to provide an enteric coating.

Other additives and excipients may then be added to the formulation of the partially water soluble carrier-active odd-chain fatty acids mixture, e.g., adding Povidone (e.g., Povidone 30), Xantham gum (or other gums) and Sorbitol to a mixture of Kaolin Clay to provide a specific example of one formulation of the present disclosure. As will be apparent to those of skill in the art, the actual amount of the partially-excipient soluble active salt (e.g., non or partially water soluble) may be varied in accordance with the dissolution characteristics of the active, which may be further varied by addition of agents that affect the solubility and/or dissolution of the active in, e.g., water. As regards a pediatric formulation, the amount of active may be reduced in accordance with the dosage form approved for pediatric use.

One example of a liquid odd-chain fatty acid(s) pharmaceutical composition may be prepared for enteral or parenteral use with the following components:

| Components | Weight |
|---|---|
| Odd-chain fatty acid(s)/triglyceride | 1.0 Kg |
| emulsifier (e.g., Imwitor 375) | 100 gr |
| Purified water (USP) | 2.0Kg |

The formulation may further include, e.g.:

| | |
|---|---|
| Glycerin (USP) | 500.0 ml |
| Sorbitol Solution, 70% (USP) | 500.0 ml |
| Saccharin Sodium (USP) | 10.0 gr |
| Citric Acid (USP) | 10.0 gr |
| Sodium Benzoate (NF) | 6.0 gr |
| Kollidon 30 | 330.0 gr |
| Xanthan Gum 200 Mesh | 20.0 gr |
| Bubble Gum Flavor | 11.1 gr |
| Methylparaben | 1.0 gr |
| Proplyparaben | 100 mg |
| Propylene Glycol (USP) | 75 ml |
| Additional ddH₂O | QS to 5 liters. |

With appropriate increases of the above for scale-up.

A batch of mixed release odd-chain fatty acids in an enveloped preparation on a carrier, e.g., beads, may be prepared with the following components:

| Components | Weight |
|---|---|
| Emulsified odd-chain fatty acids/triglyceride | 8.0mg |
| Carrier | 51.7mg |
| Calcium Stearate | 4.0mg |
| Talc | 4.0mg |
| Pharmaceutical Glaze | 5.5mg |

When combining odd-chain fatty acids (C5, C7, C9, C11, C13 and/or C15), these may be formulated as follows. A capsule for extended release of a first active and extended release of a second active in an enveloped formulation, in a single capsule:

| First Bead | Weight | Second Bead | Weight |
|---|---|---|---|
| odd-chain fatty acid C7 | 6.0 mg | odd-chain fatty acid C15 | 2.0 mg |
| Bead | 162.9 mg | Bead | 108.5 mg |
| Lacquer | 6 mg | Lacquer | 3.3 mg |
| Talc | 12.6 mg | Talc | 5 mg |
| Calcium Stearate | 12.6 mg | Calcium Stearate | 5 mg |
| Capsule | 1 | | |

When combining the odd-chain fatty acids, these may be formulated as follows. A capsule for extended release of a first active and extended release of a second active in an enveloped formulation, in a single capsule:

| First Bead | Weight | Second Bead | Weight |
|---|---|---|---|
| odd-chain fatty acid C9 | 6.0 mg | odd-chain fatty acids C11 | 2.0 mg |
| Bead | 162.9 mg | Bead | 108.5 mg |
| Lacquer | 6 mg | Lacquer | 3.3 mg |
| Talc | 12.6mg | Talc | 5mg |
| Calcium Stearate | 12.6 mg | Calcium Stearate | 5 mg |
| Mini-capsule | 1 | | |

A formulation for extended release of odd-chain fatty acids of a second active in an enveloped formulation, in a gelcap:

| Component | Weight | Component | Weight |
|---|---|---|---|
| odd-chain fatty acid C13 | 6.0 mg | odd-chain fatty acid C15 | 2.0 mg |
| Bead | 162.9 mg | Bead | 108.5 mg |
| Lacquer | 6 mg | Lacquer | 3.3 mg |
| Talc | 12.6mg | Talc | 5 mg |
| Calcium Stearate | 12.6 mg | Calcium Stearate | 5 mg |
| Gelcap | 1 | | |

A formulation for rectal release of odd-chain fatty acids in a suppository:

| Component | Weight |
|---|---|
| Odd-chain fatty acids | 100 mg |
| Carrier | 10 mg |
| Talc | 12.6 mg |
| Calcium Stearate | 12.6 mg |
| beeswax/glycerol | 1-2 gr |

An enteric-coated soft gelatin capsule that includes the odd-chain fatty acids (with or without an emulsifier) is made by coating the odd-chain fatty acids with a lipophilic material to obtain granules, mixing the granules obtained in step with an oily matrix, antioxidants and preservatives to form a lipid suspension, mixing the lipid suspension within a soft gelatin film, and coating the soft gelatin film to obtain an enteric coated soft gelatin capsule.

The odd-chain fatty acid(s), stearic acid and triethanolamine are heated and mixed to form an emulsified fluid. The resulting emulsified fluid is mixed well by a homogenizer to obtain an emulsified suspension and enterically coated. Examples of formulations include:

| Component | Weight |
|---|---|
| Odd-chain Fatty Acids | 360.0 g |
| Stearic acid | 78.6 g |
| Ethanolamine | 21.4 g |

| Component | Weight |
|---|---|
| Odd-chain Fatty Acids | 360.0 g |
| Stearic acid | 30.0 g |
| Triethanolamine | 20.0 g |

| Component | Weight |
|---|---|
| Odd-chain Fatty Acids | 400.0 g |
| Stearic acid | 77.0 g |
| Ethanolamine | 23.0 g |
| Cetyl alcohol | 50.0 g |

| Component | Weight |
|---|---|
| Odd-chain Fatty Acids | 245.0 g |
| Stearic acid | 38.5 g |
| Ethanolamine | 11.5 g |
| Cetyl alcohol | 50.0 g |
| Carboxymethyl cellulose | 25.0 g |

Alzheimer's disease (AD) is the most common of many types of dementia and is clinically characterized by severe cognitive impairment, memory loss, anomia, apraxia, and personality changes. AD is also characterized by two major neuropathological changes that can only be determined by postmortem examination. The first is diffuse plaques that consist of aggregates of a peptide called amyloid-β (Aβ)^{[1,2]}. These AP plaques are found in specific brain regions and occur as extracelluar deposits. The second is aggregates of a phosphorylated form of Tau protein that make up neurofibrillary tangles, which accumulate intraneuronally^{[3,4]}. Other changes in the AD brain including inflammatory responses and oxidative stress may also ensue and the combined effect is severe neuronal and synaptic dysfunction. The amyloid cascade hypothesis was first proposed by Hardy and Higgins in 1992 ^{[5]} and postulates that AP triggers the development of AD and other phenotypic changes, including Tau pathology. This hypothesis is supported by studies showing that in familial AD all mutations result in increased AP accumulation. In sporadic AD other underlying causes of unknown origin result in that same cascade of events. The greatest proportion of all AD cases (90 - 95%) are late-onset and sporadic in origin.

The statistics for the prevalence of AD in the aging population is alarming. Every 72 seconds someone in the United States develops this disease. An estimated 5.1 million Americans had AD in 2007 (Alzheimer's Report Facts and Figures, 2007). This number includes 200,000 individuals below 65 years with early onset AD. The proportion of Americans with AD is as follows: 2% between age 65 - 74 years; 19% between the age 75 - 84 years; and 42% in ages 85 years and above. Even more staggering is the prediction that in 2050 the number of individuals age 65 and over with AD could range from 11 to 16 million. Alzheimer's is the fifth leading cause of death in people over the age of 65. From 2000 - 2004 deaths from AD increased by 32.8%, while for other major disorders such as heart disease it decreased by 8% (CDC, National Vital Statistics Reports). These statistics are the realization that despite a tremendous amount of research that has advanced our knowledge of the risk factors and biochemical mechanisms involved in the etiology of AD there are still currently no disease-modifying therapies. At best, FDA approved drugs temporarily slow worsening of the symptoms for about 6 - 12 months in approximately half of those who take them, and current therapies are directed at improving quality of life.

Identifying the initiator of the pathophysiological cascade is crucial in finding an effective treatment. Several theories have been proposed including increased deposition of Aβ, Tau phosphorylation, oxidative stress, metal ion dysregulation, and inflammation. Despite the fact that these aspects are associated and almost certainly play a role in AD, no single one of these theories can explain the spectrum of abnormalities in this disease. However, dysregulation of mitochondrial energy metabolism may be an initiator for the major known neuropathological events. The human brain is one of the most metabolically active organs in the body. It derives its energy in the form of ATP from glucose for which it is largely dependent on uptake from the circulation. There is only a small amount of glycogen stored in the brain and it is estimated that this is sufficient to only provide enough glucose for approximately 5 minutes of normal function^{[6]}. A reproducible and consistent finding is that the cerebral metabolic rate (CMRglc) is decreased in the AD brain by 17 -24% ^{[7]}. Furthermore, low CMRgIc has been shown to be region specific and correlate with cognitive scores. It is also known that low CMRgIc appears early in the disease well before large amounts of Aβ develop. This has led to the speculation that deficits in energy metabolism precede the cascade of events initiated by Aβ. Interestingly, inhibition of energy production in a cell culture model resulted in an 80 fold increase in amyloid precursor protein (APP) that can form Aβ ^{[8]}. Energy deprivation was induced in wildtype mice treated with insulin and 2-deoxyglucose and this resulted in an increase in the activity of 13-secretase (BACE), the rate-limiting step for Aβ formation ^{[9]}. This effect lasted for 7 days and resulted in a 2 fold increase in Aβ levels. In AD the cause of the energy deficit in the brain is unclear.

Of special interest is the recent demonstration that mutations in the genes that lead to increased Aβ and phosphorylated Tau protein in transgenic mouse models are associated with mitochondrial dysfunction leading to synaptic dysfunction and apoptotic cell death suggesting a responsible secondary energy deficit. Mitochondrial dysfunction includes impairment of mitochondrial oxidative phosphorylation (complexes I, III, and IV), and reduced activities of cytochrome c oxidase, pyruvate dehydrogenase (PDH), isocitrate dehydrogenase (mICDH), and a-ketoglutarate dehydrogenase (aKGDH) all associated with decreased levels of ATP. This compromise of energy metabolism has been demonstrated in many studies of neuronal cultures exposed to the amyloid β 1-42 fragment produced by the action of β-secretase. Compromise of mitochondrial energetics in neurons would clearly lead to synaptic dysfunction and cell death associated with the progression of the disease.

In summary an early energy deficit may initiate the Amyloid cascade leading to deposition of Aβ and phosphorylated Tau proteins. These proteins may in turn act to inhibit enzymes involved in energy metabolism resulting in energy deprivation that is harmful to neurons and can contribute to the pathophysiology of AD. Improving energy metabolism may be of great benefit in the AD patient.

The use of Ketogenic diets: Ketogenic diets have been used to provide an alternative source of energy to the brain. Providing the body with a high fat, adequate protein and low carbohydrate diet mimics aspects of starvation and forces the body to burn fat rather than carbohydrate. This results in an increase in circulating ketone bodies (KB), which are taken up by the brain. This results in an increase in circulating ketone bodies (KB: β-hydroxybutyrate [BHB] and acetoacetate [AcAc]), which are taken up by the brain for energy metabolism via the citric acid cycle and the consequent ATP production via the respiratory chain. Ketogenic diets have been used to treat a variety of neurological disorders. It is also a potential therapy for AD and has been used in one transgenic animal model of AD. Van der Auwera and coworkers ^{[10]} treated mice that over-express APP and Aβ with a ketogenic diet for 43 days. These mice develop Aβ deposits in the brain as early as 3 months and have extensive deposition by 12 to 14 months. The ketogenic diet led to a substantial increase in serum BHB and a significant 25% reduction in Aβ levels in the brain. Despite these encouraging preclinical results studies of ketogenic diets in AD are lacking. Only one study has been published on the use of medium chain triglycerides (MCT) in probable AD subjects ^{[11]}. Twenty AD subjects were given a single 40 g dose of MCT and this resulted in significant improvement in cognition (ADAS-cog) scores after 90 minutes and a 10 fold increase in serum BHB concentration ^{[11]}. The authors conclude that achievable levels of ketosis may be beneficial to the AD patient.

An alternative and more efficient means of providing an energy source to the brain is by supplementation with triheptanoin (C7TG) as disclosed herein. When given enterally, this triglyceride is first converted to one mole of glycerol and 3 moles of heptanoate (C7) that are delivered to the liver via the portal circulation. Heptanoate enters the mitochondria where C7 is converted to C5-CoA (pentanoyl-CoA), then 3-keto-pentanoyl-CoA (BKPCoA). BKP-CoA is then further metabolized via cleavage to acetyl-CoA and propionylCoA thus fueling the CAC in liver, directly, but also BKP-CoA enters the HMG pathway producing the C5-ketone bodies (3-hydroxypentanoate and 3-ketopentanoate) that are exported to other organ systems including brain where they are again converted to propionyl- and acetyl-CoA providing needed substrate for the CAC ^{[12]} and enhanced ATP generation. C7TG has been used successfully to treat patients with inherited disorders of fat and glycogen metabolism as well as pyruvate carboxylase deficiency with positive effects on indirect measures of energy metabolism in the brain and peripheral organs such as muscle and heart ^{[13, 14]}. Early correction of an energy deficit in AD is key to a successful disease-modifying therapy. It is clear that early intervention is crucial to prevent the cascade of events triggered by Aβ. Once the amyloidgenic plaques have been deposited and phosphorylated-Tau has developed into neurofibrillary tangles in brain tissue, irreversible damage to the neuron and synapse takes place. Increasing energy metabolism at a pre-symptomatic stage of AD may prevent the development of the full-blown pathology.

Prior Benefits and Mechanism of action for Dietary Triheptanoin: The benefits of C7 -TG are now understood as a result of both animal and human clinical trials. The treatment hypothesis is based on the likelihood that energy metabolism is seriously compromised by the inability to adequately fuel the citric acid cycle (CAC) in subjects with these inherited disorders. For effective function of the CAC, linked to the respiratory chain for ATP production, adequate oxaloacetate along with acetyl-CoA is required for the citrate synthase reaction. Since glucose and medium-chain fatty acids (C8 and CIO) can only provide acetyl-CoA, the effect of C7-TG is a superior source of both acetyl-CoA and oxaloacetate (derived from the propionyl-CoA moiety) both of which are produced inside the mitochondrion and provide the necessary substrates to fuel the CAC. When given enterally, heptanoate (C7) derived from C7-TG is almost totally taken up by the liver. C7 does not require CPT I, carnitine-acylcamitine translocase or CPT II for entry into the mitochondrion. It appears to be activated to heptanoyl-CoA by the medium-chain acyl-CoA synthetase. After one cycle of β-oxidation acetyl-CoA and pentanoyl-CoA are produced. The latter is then oxidized to β-ketopentanoyl- CoA that can undergo thiolytic cleavage to produce another acetyl-CoA moiety and propionyl-CoA. The two acetyl-CoA produced can be converted to acetoacetyl-CoA. Acetoacetyl-CoA and β-ketopentanoyl-CoA can both proceed via the HMG-CoA pathway forming ketone bodies containing both 4 (acetoacetate and β-hydroxybutyrate) and 5 carbons (β-ketopentanoate [BKP] and β-hydroxypentanoate [BHP]). When exported from the liver all peripheral organs including brain take up both sets of ketone bodies. As with acetoacetate and β-hydroxybutyrate, the ketone utilizing enzymes in the mitochondria of other organs activate both BKP and BHP to CoA thioesters. BHP-CoA is converted to BKP-CoA that is cleaved to both acetylCoA and propionyl-CoA. Propionyl-CoA enters the CAC at the level of succinyl-CoA and is converted to oxaloacetate. Both acetyl-CoA and oxaloacetate are then available for the citrate synthase reaction in the CAC. The result is increased ATP formation via the respiratory chain potentially correcting the energy deficit. This is the basis of "anaplerotic" therapy- direct fueling of the CAC by providing needed substrate.

In addition to benefits for congestive heart failure, hypertrophic and dilated cardiomyopathies, hepatic failure, and disorders associated with recurrent rhabdomyolysis and hypoglycemia, rapid and dose dependent responses have been observed in several CNS deficits. These have included settings that would otherwise be labeled "autism", and dementias similar to Alzheimer's. Progressive loss of name recall, ability to focus and concentrate for reading, "knowing" what to say but the words won't come, and repetition of conversations without knowledge that it was said only 5 minutes before, are all cognitive deficits associated with early-onset Alzheimer's disease. C7 -TG and other odd-chain fatty acid compounds, as described herein, may be used to treat older subjects with senile dementia and early Alzheimer's disease in an attempt to offset or delay the possible metabolic effects operating in senescence.

Rationale for the use of Tripentanoin (Glyceryl-tripentanoate-CSTG): As described above, pentanoyl-CoA is an intermediate of hepatic oxidation of heptanoate derived from C7TG. Further metabolism of pentanoyl-CoA is identical to that of heptanoate but requires fewer steps to achieve the same end-result of providing acetylCoA and propionyl-CoA for liver energy production and also the 5-carbon ketone bodies (BKP and BHP) for export to the other organ systems. Whereas heptanoate donates 2 moles of acetyl-CoA and 1 mole of propionyl-CoA, pentanoate provides only one mole of each. This reduces production of 4-carbon ketone bodies (BHB and AcAc) while favoring production of the 5-carbon ketone bodies (BKP and BHP). This eliminates the competition for utilization of BKP and BHP thus providing more effective anaplerosis from pentanoate. Enhanced anaplerosis from pentanoate (stimulation of the CAC and energy production) in mouse brain following infusion of propionyl-CoA precursors has been clearly demonstrated to be superior to heptanoate, BKP, or propionate^{[15]}. For the above reasons, the present invention utilizes enteral tripentanoin to provide optimal anaplerosis to the brain of these mouse models.

There is a late life onset alteration that shifts the degradation of AbPP from the non-amyloidogenic pathway to the amyloidogenic pathway, possibly related to mutations or modification of activities of the α- and β-secretase complexes. This may also be related to the insidious impact of beta amyloid on mitochondrial function in energy metabolism. Previously demonstrated mitochondrial dysfunction in Alzheimer's disease may be associated with deficit of substrates for the CAC that can cause an ATP deficit as well as an intra-mitochondrial redox alteration leading to synaptic dysfunction and cell death. Since prior studies show the same problem of AbPP degradation (platelets, etc.) and the possibility that peripheral organs may have the same accumulations of beta amyloid there is increasing evidence that beta amyloid may also come from peripheral organ metabolism to the brain and be incorporated intra-cellularly where it impacts on mitochondrial function leading to neuronal cell death. Tripentanoin (C5-TG), via its hepatic oxidation may produce sufficient "S-carbon ketone" bodies to provide the brain with adequate substrate that may offset the energy deficit in this disease. Treatment of both the "peripheral" organs and the CNS seems important to alleviate the effects of beta amyloid or its enhanced production on the neurons involved in Alzheimer's disease and possibly other situations of elderly onset dementia that may be the result of impaired energy production in the brain. A novel variation of "ketogenic" diet therapy - fueling the citric acid cycle directly with both acetyl-CoA and propionyl-CoA. The desired result would be normalization of the redox abnormalities, normal functioning of PDH, mICDH, and uKGDH, and enhanced ATP production. The disclosure outlines studies directed to study the effect of C5TG in transgenic animal models that recapitulate some of neuropathological changes that occur in the AD brain.

The present disclosure describes studies directed towards: (i) determining the effect of C5-TG on cognitive function, locomotor activity and open field behavior in an AD double transgenic mouse which over-expresses mutant human APP and presenilin, (ii) determining the effect of C5-TG on cognitive function, locomotor activity and open field behavior in a transgenic mouse in over-expresses microtubule associated phopshorylated Tau (MAPT) in the absence of other factors including AP, (iii) determining the effect of C5-TG on cognitive function, locomotor activity and open field behavior in an C57BL16 and 22 month old mice, and (iv) assessing neurochemical and metabolic changes in AD and aging mouse models treated with C5-TG. Quantitative analysis in regional brain tissue includes: AP fragments 1-40 and 1-42, phosphorylated-Tau, high energy phosphate compounds (AMP/ADP/ATP), Acetylcholine (Ach), dopamine (DA), serotonin (5HT), gammahydroxybutyrate (GABA) and full amino acid analysis. Plasma amino acids, acyl-carnitines, amino acids, and urine organic acids are also determined.

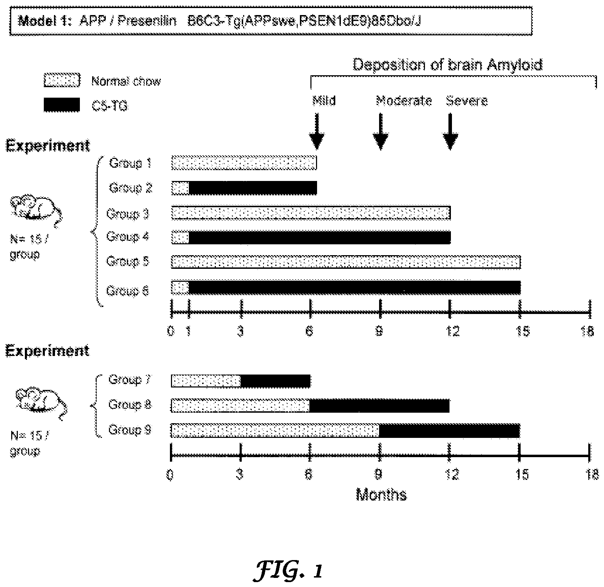

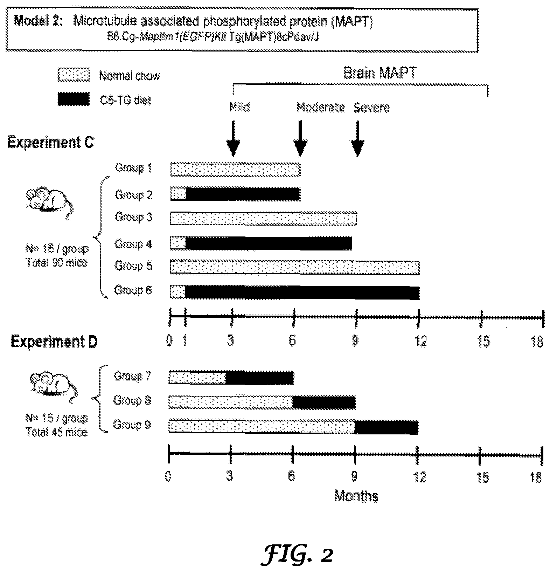

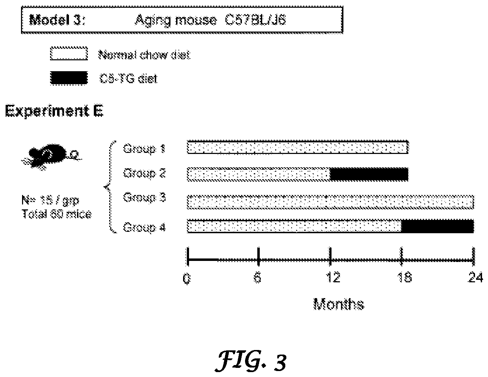

Rationale for mouse models: The efficacy of C5-TG is tested in two transgenic AD models and an aging mouse model. In Model 1 **(****FIG. 1****)** mice that over-express APP and AP is evident at 6 months with extensive deposition at 12 months. Cognitive impairments are also evident as early as 6 months and become more pronounced with age. In Model 2 **(****FIG. 2****)** transgenic mouse that over-express microtubule associated phosphorylated Tau (MAPT) in the absence of other factors including AP was studied. This pathogenic Tau accumulates in the cell bodies and dendrites of neurons in a spatiotemporally relevant distribution. In Model 3 **(****FIG. 3****)** aged mice are studied. Aged mice have been previously characterized at Baylor Institute of Metabolic Disease. At 22 months of age there is a significant decrease in locomotive activity and cognitive function assessed by the Morris Water Maze, compared to 3 month old mice of the same strain. This model is used to examine the effect of C5-TG on age related cognitive decline. In humans this is equivalent to mild cognitive impairment (MCI) of which approximately 40% of patients progress to AD. These models and availability are described in detail below.

Model 1: AD transgenic mouse: Jackson Laboratories (Stock number 004462), Strain Name: *B6C3-Tg(APPswe,PSENldE9)85Dbo*/*J,* Double transgenic mice express a chimeric mouse/human amyloid precursor protein *(Mo*/*HuAPP695swe)* and a mutant human presenilin 1 (PS 1-dE9) both directed to CNS neurons. Both mutations are associated with early-onset Alzheimer's disease. The "humanized" *Mo*/*HuAPP695swe* transgene allows the mice to secrete a human A-beta peptide. Both the transgenic peptide and holoprotein can be detected by antibodies specific for human sequence within this region (Signet Laboratories' monoclonal 6E10 antibody). The included Swedish mutations *(K595N*/*M596L)* elevate the amount of A-beta produced from the transgene by favoring processing through the beta-secretase pathway. This "humanized" *Mo*/*HuAPP695swe* protein is immunodetected in whole brain protein homogenates. The transgenic mutant human presenilin protein (PS1-dE9), which in high levels displaces detectable endogenous mouse protein, is also immunodetected in whole brain protein homogenates. These transgenic mice develop beta-amyloid deposits in brain by six to seven months of age and impairment in cognitive function. These mice may be useful in studies of neurological disorders of the brain, specifically Alzheimer's disease, amyloid plaque formation, and aging.

Model 2: AD transgenic mouse: Jackson Laboratories (Stock number: 05491), Strain Name: *B6.Cg_Maplml(EGFP)Klt Tg(MAPT)8cPdav*/*J* Mice that are homozygous for the targeted allele and hemizygous for the trans gene are viable and fertile. Although no endogenous mouse MAPT is detected, all six isoforms (including both 3R and 4R forms) of human MAPT are expressed. Hyperphosphorylated MAPT is detected in cell bodies and dendrites by three months of age. Paired helical filaments of aggregated insoluble MAPT can be isolated from brain tissue as early as two months of age. These mutant mice may be useful in studies examining the relationship between human MAPT and Alzheimer's disease pathogenesis

Model 3: Aging mouse: NIH/NIA (maintained at Charles River Labs), Strain: C57BL16 NIA maintains colonies of aged rats and mice for use by the scientific community for research directly related to aging and age-related diseases. The animals are housed behind specific pathogen-free barriers and monitored for genetic purity and health status, and a health report accompanies each shipment of animals. At 22 months of age there is a significant decrease in locomotive activity and cognitive function assessed by Rotarod and Morris Water Maze testing, compared to 3 month old mice of the same strain.

Study Design: The mice are housed at the animal facility at Baylor Institute of Metabolic disease. Test diets containing C5-TG and control diets are specially prepared from a standard casein purified diet (Harland Teklad, MN). In the test diet 41 % of the total caloric requirement is supplied by C5-TG. In Models 1 and 2 (Tg AD)(**FIGS. 1** and **2**) mice are exposed to the diets starting at 4 weeks of age and maintained for a period of either 6, 12 or 15 months. Separate groups of animals are required for behavioral, neurochemical, and metabolic testing at 3 month intervals. The objective of the study was to initiate treatment with C5-TG in mice prior to any neuropathogenic changes and assess cognitive function. In another series of studies mice are exposed to the C5-TG test diets starting at either 6 or 12 months of age. Mice were assessed at 3 month intervals up until 18 months of age. The objective of this study is to determine the effect of C5-TG in modifying disease progression after neuropathogenic changes in the brain have been established.

In Model 3 (**FIG. 3**) the mice are exposed to C5-TG and control diets at 12 months of age and assessed for behavioral and neurochemical changes at 18 and 24 months of age. The objective of this study is to determine the effect of C5-TG on age related cognitive decline in the absence of any abnormally expressed AP or phosphorylated-Tau protein.

Behavioral methods: The following behavioral methods were established and validated in the Neuropharmacolgy laboratory at Baylor Institute of Metabolic Disease.
1. Morris Water Maze test for temporal-spatial memory function.
2. RotaRod performance test for motor coordination.
3. Open-Field behavioral testing in photobeam activated arenas to assess exploratory behavior, locomotor activity and anxiety.
4. Grip strength testing of rear and hind legs.

Neurochemical Methods: The following neurochemical methods were established and validated in the Neuropharmacology laboratory.
1. Amyloid β fragments 1-40 and 1-42 in brain homogenates were measured using ELISA test kits.
2. Phospho-Tau protein in brain homogenates are determined using ELISA test kits.
3. Brain AMP/ ADP / ATP measurements in regional brain tissues were measured by HPLC with UV detection. Mice required sacrificing by microwave fixation to inactivate enzymes and prevent post-mortem changes.
4. Brain Acetylcholine (Ach) measurements in regional brain tissues were measured by HPLC with electrochemical detection. Mice required sacrificing by microwave fixation to inactivate enzymes and prevent post-mortem changes.
5. Brain dopamine, serotonin and norepinephrine regional brain tissues were measured by HPLC with electrochemical detection.
6. Full quantitative amino acid profiling in plasma and brain tissue was done by HPLC with UV detection. GABA and other excitatory and inhibitory amino acids were determined in brain tissue.
7. Urine organic acids and blood acyl-carnitines were determined by GC-MS.

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "or combinations thereof' as used herein refers to all permutations and combinations of the listed items preceding the term. For example, "A, B, C, or combinations thereof is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, MB, BBC, AAABCCCC, CBBAAA, CABABB, and so forth. The skilled artisan will understand that typically there is no limit on the number of items or terms in any combination, unless otherwise apparent from the context.

### REFERENCES

United States Patent No. 6,335,361: Method of treating benign forgetfulness.
United States Patent Application No. 20080287372: Use of Ketogenic Compounds for Treatment of Age-Associated Memory Impairment.
United States Patent Application No. 20090253781: Therapeutic compositions.
   1. Glenner GG, Wong CW, Quaranta V, Eanes ED. The amyloid deposits in Alzheimer's disease: their nature and pathogenesis. Appl Pathol. 1984;2(6):357-69.
   2. Masters CL, Simms G, Weinman NA, Multhaup G, McDonald BL, Beyreuther K. Amyloid plaque core protein in Alzheimer disease and Down syndrome. Proc Nat! Acad Sci USA. 1985;82( 12):4245-9.
   3. Goedert M, Wischik CM, Crowther RA, Walker JE, Klug A. Cloning and sequencing of the cDNA encoding a core protein of the paired helical filament of Alzheimer disease: identification as the microtubule-associated protein tau. Proc Natl Acad Sci USA. 1988; 85(11):4051-5
   4. Grundke-Iqbal I, Iqbal K, Tung YC, Quinlan M, Wisniewski HM, Binder LI. Abnormal phosphorylation of the microtubule-associated protein tau (tau) in Alzheimer cytoskeletal pathology. Proc Nat! Acad Sci USA. 1986;83(13):4913-7.
   5. Hardy JA, Higgins GA. Alzheimer's disease: the amyloid cascade hypothesis. Science. 1992 Apr 10;256(5054): 184-5. Review.
   6. Clarke DO, Sokoloff L. Circulation and energy metabolism of the brain. In: Siegel GJ, AgranoffBW, Albers RW, MolinoffPB, eds. Basic neurochemistrt. New York: Raven Press, 1994: 645-680.
   7. Buckner RL, Andrews-Hanna JR, Schacter DL. The brain's default network: anatomy, function, and relevance to disease. Ann NY Acad Sci. 2008;1124:1-38. Review.
   8. Gabuzda 0, Busciglio J, Chen LB, Matsudaira P, Yankner BA. Inhibition of energy metabolism alters the processing of amyloid precursor protein and induces a potentially amyloidogenic derivative. J Biol Chern. 1994;269(18): 13623-8
   9. Velliquette RA, O'Connor T, Vassar R. Energy inhibition elevates beta-secretase levels and activity and is potentially amyloidogenic in APP transgenic mice: possible early events in Alzheimer's disease pathogenesis. J Neurosci. 2005;25(47):10874-83.
   10. Van der Auwera I, Wera S, Van Leuven F, Henderson ST. A ketogenic diet reduces amyloid beta 40 and 42 in a mouse model of Alzheimer's disease. Nutr Metab (Lond). 2005;2:28.
   11. Reger MA, Henderson ST, Hale C, Cholerton B, Baker LD, Watson OS, Hyde K, Chapman D, Craft S. Effects of beta-hydroxybutyrate on cognition in memoryimpaired adults. Neurobiol Aging. 2004;25(3):311-4.
   12. Kinman RP, Kasumov T, Jobbins KA, Thomas KR, Adams JE, Brunengraber LN, Kutz 0, Parenteral and enteral metabolism of anaplerotic triheptanoin in normal rats. Brewer WU, Roe CR, Brunengraber H. Am J Physiol Endocrinol Metab. 2006;291(4):
   13. Roe CR, Mochel F. Anaplerotic diet therapy in inherited metabolic disease: therapeutic potential.J Inherit Metab Dis. 2006;29(2-3):332-40. Review.
   14. Roe, C.R., Sweetman, L., Roe, D.S., David, F., Brunengraber, H.Effective Dietary Treatment of Cardiomyopathy & Rhabdomyolysis in Long-Chain Fat Oxidation Disorders using an Anaplerotic Odd-Chain Triglyceride. J. Clin. Invest. 110 (2): 259-269, 2002.
   15. Xiao Wang, Guofang Zhang, Michelle A. Puchowicz ,Takhar Kasumov, Frederick Allen Jr, Adam J. Rubin, Ming Lu, Xin Yu , Charles R. Roe, and Henri Brunengraber: Fatty acid oxidation and anaplerosis from (Intravenous) propionylCoA precursors in normal and the very-long-chain acyl-CoA dehydrogenase deficient mouse brain. J. BioI. Chern. In Press 2009.

## Claims

1. A formulation for use in treating an adult patient suffering from Alzheimer's disease (AD) comprising the step of:
administering the formulation to the patient in a quantity sufficient to treat or alleviate the symptoms of Alzheimer's disease (AD), wherein the formulation comprises triheptanoin.

2. The formulation for the use according to claim 1, wherein the formulation comprises one or more optional additives selected from the group consisting of flavoring agents, vitamins, mineral supplements, protein supplements, coloring agents, and preservatives.

3. The formulation for the use according to claim 1, wherein upon administration the formulation increases a level of one or more circulating ketone bodies in the blood of the human subject.

4. The formulation for the use according to claim 1, wherein the formulation is adapted for parenteral, enteral, intravenous, or intramuscular administration.

5. The formulation for the use according to claim 1, wherein the triheptanoin is provided in an amount suitable for providing 35% kcalories of the daily dietary caloric requirement for adults.

6. The formulation for the use according to claim 1, wherein the triheptanoin is provided in an amount suitable for administration of 0.1-2 g/kg body weight of adults.

## Patentansprüche

1. Formulierung zur Verwendung bei der Behandlung eines erwachsenen Patienten, der an der Alzheimer-Krankheit (AK) leidet, folgenden Schritt umfassend:
Verabreichen der Formulierung an den Patienten in einer Menge, die ausreichend ist, die Symptome der Alzheimer-Krankheit (AK) zu behandeln oder zu lindern, wobei die Formulierung Triheptanoin umfasst.

2. Formulierung zur Verwendung nach Anspruch 1, wobei die Formulierung ein oder mehrere optionale Additive umfasst, die aus der Gruppe ausgewählt sind, die aus Aromastoffen, Vitaminen, Mineralstoffergänzungsmitteln, Eiweißergänzungsmitteln, Farbstoffen und Konservierungsmitteln besteht.

3. Formulierung zur Verwendung nach Anspruch 1, wobei die Formulierung nach der Verabreichung einen Spiegel eines oder mehrerer zirkulierender Ketonkörper im Blut des menschlichen Subjekts erhöht.

4. Formulierung zur Verwendung nach Anspruch 1, wobei die Formulierung für eine parenterale, enterale, intravenöse oder intramuskuläre Verabreichung eingerichtet ist.

5. Formulierung zur Verwendung nach Anspruch 1, wobei das Triheptanoin in einer Menge bereitgestellt ist, die geeignet ist, 35 % der Kilokalorien des täglichen Kalorienbedarfs eines Erwachsenen aus der Nahrung bereitzustellen.

6. Formulierung zur Verwendung nach Anspruch 1, wobei das Triheptanoin in einer Menge bereitgestellt ist, die zur Verabreichung von 0,1 bis 2 g/kg des Körpergewichts von Erwachsenen geeignet ist.

## Revendications

1. Formulation destinée à une utilisation pour traiter un patient adulte souffrant de la maladie d'Alzheimer (MA), comprenant l'étape consistant à :
administrer la formulation au patient en une quantité suffisante pour traiter ou atténuer les symptômes de la maladie d'Alzheimer (MA), ladite formulation comprenant de la triheptanoïne.

2. Formulation destinée à l'utilisation selon la revendication 1, ladite formulation comprenant un ou plusieurs additifs facultatifs choisis dans le groupe constitué par des agents aromatisants, des vitamines, des compléments minéraux, des compléments protéiques, des agents colorants et des conservateurs.

3. Formulation destinée à l'utilisation selon la revendication 1, ladite formulation augmentant lors de son administration un niveau d'un ou plusieurs corps cétoniques circulants dans le sang du sujet humain.

4. Formulation destinée à l'utilisation selon la revendication 1, ladite formulation étant adaptée pour l'administration par voie parentérale, entérale, intraveineuse ou intramusculaire.

5. Formulation destinée à l'utilisation selon la revendication 1, la triheptanoïne étant fournie en une quantité adéquate pour fournir 35 % des kilocalories du besoin calorique alimentaire journalier pour des adultes.

6. Formulation destinée à l'utilisation selon la revendication 1, la triheptanoïne étant fournie en une quantité adéquate pour l'administration de 0,1 à 2 g par kg de masse corporelle des adultes.
